# EUROPÄISCHE PATENTSCHRIFT

(11) **EP 2 042 085 B1**
(45) Veröffentlichungstag und Bekanntmachung des Hinweises auf die Patenterteilung: **19.12.2018**
(21) Anmeldenummer: 08016968.3
(22) Anmeldetag: 26.09.2008
(51) Int. Cl.: A61B 5/00

(54) **Verfahren zur Messung eines mindestens eine semitransparente Schicht aufweisenden Körpers**
Method for measuring a body with at least one semitransparent layer
Procédé de mesure d'au moins un corps comportant une couche semi-transparente

(30) Priorität: 26.09.2007 DE 102007046228
(43) Veröffentlichungstag der Anmeldung: 01.04.2009
(73) Patentinhaber: DENTSPLY SIRONA Inc., York, PA 17401-2991 (US)
(72) Erfinder: Ertl, Thomas, 63303 Dreieich (DE)
(74) Vertreter: Stoffregen, Hans-Herbert

(56) Entgegenhaltungen:
- US-A- 4 589 846
- US-A- 4 836 206
- US-A1- 2007 099 148
- US-A1- 2007 134 615
- DATABASE MEDLINE [Online] US NATIONAL LIBRARY OF MEDICINE (NLM), BETHESDA, MD, US; September 2004 (2004-09), PRETTY IAIN A ET AL: "A closer look at diagnosis in clinical dental practice: part 5. Emerging technologies for caries detection and diagnosis.", Database accession no. NLM15363214 & PRETTY IAIN A ET AL: "A closer look at diagnosis in clinical dental practice: part 5. Emerging technologies for caries detection and diagnosis.", JOURNAL (CANADIAN DENTAL ASSOCIATION) SEP 2004, vol. 70, no. 8, September 2004 (2004-09), pages 540 , 540a-540i, ISSN: 1488-2159
- PETER SCHÄFER: "Kariesdiagnostik mit Ditofi", , 29 May 2007 (2007-05-29), Retrieved from the Internet: URL:https://edoc.ub.uni-muenchen.de/6838/1 /Schaefer_Peter.pdf> [retrieved on 2017-09-27]

## Beschreibung

Die Erfindung bezieht sich auf ein Verfahren zum Erkennen von Veränderungen an zumindest einem Zahn im Dentin- und/oder Zahnschmelzbereich, insbesondere zum Erkennen von Karies oder Zahnschmelzriss, durch Beaufschlagen des Zahnes mit aus zumindest einem Lichtleiter über dessen Stirnfläche austretendem Licht sowie Erfassen zumindest eines Bereichs des zumindest einen Zahns mittels eines optischen Sensors, wie Kamera, und anschließendes Auswerten von von dem Sensor ermitteltem Bild bzw. ermittelten Bildern, wobei zum Beaufschlagen des zumindest einen Zahns mit dem Licht des zumindest einen Lichtleiters die Stirnfläche des Lichtleiters auf den Zahn aufgesetzt oder im Abstand a mit a ≤ 2d mit d = Lichtleiterkerndurchmesser zur Oberfläche des Zahns derart positioniert wird, dass von der Zahnoberfläche reflektiertes Licht ausschließlich in den Lichtleiter hinein zurückreflektiert wird, und wobei mit dem optischen Sensor aus dem Zahn austretendes Streulicht gemessen wird.

Zum Erkennen von Karies hat sich die Röntgendiagnostik bewährt. Da jedoch die Röntgenstrahlung immer weniger Akzeptanz bei Patienten findet, bedarf es insbesondere bei der interproximalen Kariesdiagnostik, also Karies im Approximalraum, bildgebender Alternativverfahren ohne ionisierende Strahlung. Zwar erfolgt häufig eine visuelle Diagnostik des Zahnarztes. Allerdings ist hierbei von Nachteil, dass die typischerweise kurz unterhalb des Kontaktpunktes zweier Zähne auftretende Karies, also dort, wo Zähne sich benachbart gegenüber stehen, eine Veränderung visuell erst sehr spät erkennbar ist.

Um die weitgehend subjektive visuelle Diagnostik des Zahnarztes zu vermeiden, sind nicht-bildgebende und bildgebende Verfahren bekannt.

Anordnungen zur nicht-bildgebenden Kariesdetektion beruhen derzeit auf Fluoreszenzmessungen in Reflexionsanordnung (EP 0 962 185 B1) oder Reflexionsmessungen mit verschiedenen Lichtwellenlängen ohne Nutzung von Fluoreszenzanregung (WO 03/094771). Dabei wird Licht mit einem Lichtleiter (Glasfaser oder Saphirprisma) auf die Zahnoberfläche gestrahlt und das reflektierte Licht über denselben oder einen weiteren Lichtleiter in einem Lichtempfänger geleitet. Als Lichtempfänger werden dabei typischerweise Fotodioden benutzt. Sende- und Empfangslichtleiter stehen dabei in einer koaxialen Anordnung in fester Beziehung zueinander. Je nach Zustand des Zahns (Karies / keine Karies) wird Licht unterschiedlicher spektraler Zusammensetzung detektiert. Aus dem Verhältnis der Lichtreflexion in verschiedenen Spektralbereichen oder der Fluoreszenzintensität wird auf Vorhandensein von Karies und - in engen Grenzen - Größe und Eindringtiefe der Karies geschlossen.

Nachteil der nicht-bildgebenden Verfahren ist die schlechte Interpretierbarkeit durch den Zahnarzt durch fehlende Information hinsichtlich der räumlichen Verteilung. Es wird lediglich ein numerischer Wert bereitgestellt (z. B. 0 = Gesunder Zahn, 100 = stark kariöser Zahn) oder durch verschiedene akustische Signale eine begrenzte Kariesgraddifferenzierung bereitgestellt. Die Korrelation zur histologischen Kariesausprägung ist jedoch begrenzt.

Zu den bildgebenden Verfahren gehören Transmissionsmessungen ohne Fluoreszenzanregung (WO 98/29050; US 2006/02230329A1). Dabei werden Anordnungen benutzt, die prinzipiell mit denen vergleichbar sind, die bei der Röntgendiagnostik zum Einsatz gelangen. Das transmittierte Licht einer flächigen Beleuchtung des Zahns wird auf der gegenüberliegenden Seite des Zahns von einer Kamera detektiert. Nach der WO 98/29050 wird Licht in sichtbarem Bereich des Spektrums genutzt. Demgegenüber sieht die US2006/0223032 A1 den Einsatz von Licht im nahen Infrarot vor. Dies führt zu einer deutlich verbesserten Bildqualität bei der Durchleuchtung von Zahnschmelz. Wird Licht im sichtbaren Bereich benutzt, so ist die Bildqualität nicht mit der eines Röntgenbildes vergleichbar. Daher hat sich der Einsatz von Licht im Wellenlängenbereich zwischen 1300 nm und 1400 nm bewährt. Nachteilig hierbei ist jedoch, dass ein kostenintensives InGaAs-Array als Detektor verwendet werden muss. Aus Kostengründen sind daher entsprechenden Anordnungen einer kommerziellen Nutzung für die Kariesdiagnostik Grenzen gesetzt. Des Weiteren ist von Nachteil, dass zur Vermeidung einer Überstrahlung des Sensors an Stellen, an denen sich kein Zahn oder Zahnfleisch im Strahlengang befindet, Polarisationsfilter eingesetzt werden müssen.

Des Weiteren sind Fluoreszenzmessungen in Reflexionsanordnungen bekannt. Hierbei kann das Fluoreszenzbild einer Intraoralkamera zur Kariesdiagnostik verwendet werden. Es wird in einer Reflexionsanordnung die Autofluoreszenz der Zahnhartsubstanz gemessen. Gesunde Zahnhartsubstanz, insbesondere die Dentinschicht, emittiert bei UV-Beleuchtung Licht im grünen Spektralbereich. Sind kariöse Stellen vorhanden, so sinkt die Autofluoreszenz signifikant ab. Entsprechende Verfahren sind zur Detektion von Fissuren-Karies und für Glattflächen-Karies außerhalb des Approximalraums einsetzbar. Für eine interproximale Kariesdetektion, also Karies im Approximalraum, kann aufgrund fehlender Reflexion eine Diagnostik nicht erfolgen. Unabhängig hiervon ist den bekannten bildgebenden Verfahren zur Kariesdetektion gemeinsam, dass eine weiträumige Beleuchtungsquelle genutzt wird, die den Zahn komplett oder zumindest einen größeren Teil des Zahns oder ggfs. mehrere Zähne gleichzeitig beleuchtet.

Neben den zuvor erwähnten optischen Techniken gelangen auch Impedanzmessungen zur Kariesdiagnostik zur Anwendung, die jedoch aufgrund des jeweiligen Feuchtegrades des Zahnes Nachteile bezüglich der Sensibilität zeigen.

Gegenstand der US-A-4 836 206 sind ein Verfahren und eine Vorrichtung, um die Vitalität von Zähnen zu erfassen. Hierzu wird mittels eines Lichtleiters weißes Licht einem Zahn zugeführt, um sodann eine wellenlängenabhängige Lichtabsorption des aus dem Zahn austretenden Lichts mittels Photodetektoren zu ermitteln.

Ein Scanverfahren für Zähne ist der US-A-2007/0134615 zu entnehmen.

Um Karies in einem Zahn zu ermitteln, wird dieser nach der US-A-2007/0099148 mit Licht beaufschlagt, um sodann das reflektierte Licht über Filter einer monochromatischen Kamera zuzuleiten.

Eine Anordnung zum Durchleuchten eines Zahnes, um Wurzelkanäle zu detektieren, ist aus der US-A-4 589 846 bekannt.

Der Literaturstelle "DATABASE MEDLINE [Online] US NATIONAL LIBRARY OF MEDICINE (NLM), BETHESDA, MD, US; September 2004 (2004-09), PRETTY IAIN A ET AL: "A closer look at diagnosis in clinical dental practice: part 5. Emerging technologies for caries detection and diagnosis.", Database accession no. NLM15363214; & PRETTY IAIN A ET AL: "A closer look at diagnosis in clinical dental practice: part 5. Emerging technologies for caries detection and diagnosis.", JOURNAL (CANADIAN DENTAL ASSOCIATION) SEP 2004, Bd. 70, Nr. 8, September 2004 (2004-09), Seiten 540, 540a-540i, ISSN: 1488-2159" wird ein unter der Bezeichnung DiFOTI bekanntes System beschrieben, mittels dem eine Kariesdetektion erfolgt, dabei wird auf einen Zahn ein Lichtleiter gesetzt, um innerhalb des Zahns erfolgende Reflektionen mittels eines optischen Sensors zu erfassen.

Der vorliegenden Erfindung liegt die Aufgabe zu Grunde, ein Verfahren der eingangs genannten Art so weiterzubilden, dass mit hoher Genauigkeit Veränderungen an/in zumindest einem semitransparenten Objekt insbesondere Zahn, und in diesem speziellen Fall Karies wie Fissuren-Karies oder Glattflächen-Karies, insbesondere Karies im Approximalraum erfasst werden, und dass eine hohe Bildqualität erzielbar ist, die eine Auswertung ermöglicht, die weitgehend derjenigen entspricht, die mit Röntgenstrahlung erreicht werden kann, ohne dass die Nachteile der Röntgenstrahlung selbst in Kauf genommen werden müssen.

Erfindungsgemäß wird die Aufgabe im Wesentlichen dadurch gelöst, dass zumindest ein erstes Bild des zumindest einen Zahns in Bezug auf die Veränderung bei Lichtbeaufschlagung vor der Veränderung des zumindest einen Zahns aufgenommen wird und dass zumindest ein zweites Bild des zumindest einen Zahns bei Lichtbeaufschlagung hinter der Veränderung aufgenommen wird, jeweils in Bezug zur Sensorposition, und dass das zumindest eine erste Bild mit dem zumindest einem zweiten Bild verrechnet wird.

Durch die erfindungsgemäße Lehre erfolgt eine lokal eng begrenzte Lichtinjektion in den zumindest einen Zahn, wodurch sich eine den optischen Eigenschaften des Körpers wie Zahns entsprechende Lichtverteilung im Zahnvolumen einstellt, die sodann in zumindest einer von der Lichtinjektionsstelle verschiedenen Position mit dem optischen Sensor beobachtet wird. Von der Zahnoberfläche reflektierte Strahlung gelangt in den Lichtleiter zurück, so dass Verfälschungen des Messergebnisses nicht erfolgen können.

Erfindungsgemäß wird insbesondere der geometrische Aufbau eines Zahns als Zweischichtsystem sowie die unterschiedlichen optischen Eigenschaften von Zahnschmelz und Dentin sowie deren Veränderung durch eine Veränderung des Zahns wie durch Karies oder Zahnschmelzriss genutzt.

Gesunder Zahnschmelz als äußere Hülle eines Zahns mit einer Schichtdicke von typischerweise 0,5 - 3 mm hat einen Streukoeffizienten im Bereich von ca. 0,2 /mm bis 10/mm fallend zu längeren Wellenlängen. Der Streukoeffizient von Dentin hingegen liegt im Bereich 2/mm bis 80/mm ebenfalls fallend zu längeren Wellenlängen. Der Absorptionskoeffizient von Zahnschmelz liegt im Bereich 0,0003 /mm bis 0,8 /mm und von Dentin im Bereich von 0,02/mm - 2/mm ebenfalls fallend zu längeren Wellenlängen.

Dieser Trend wird erst im Bereich um 1600 nm umgekehrt, da sodann die Wasserabsorption an Einfluss gewinnt und über lokale Maxima bei 2 µm das absolute Maximum bei 3 µm erreicht.
Für eine hohe Eindringtiefe sind daher Wellenlängen größer als 300 nm und bis zu 1600 nm zu bevorzugen. Zum Erreichen der höchsten Empfindlichkeit auf Änderungen des Streukoeffizienten und Detektion kleiner Areale mit unterschiedlichem Streukoeffizient bei Lokalisationen, bei denen keine hohe Eindringtiefe benötigt wird, bietet sich die Verwendung von violettem oder blauem Licht an. Für Fälle mittlerer Eindringtiefe entsprechende Wellenlängen zwischen diesen beiden Extremen.

Injiziert man Licht (lokal auf den Lichtleiter- wie Faserdurchmesser beschränkt) über einen auf den Zahn okklusal aufgesetzten Lichtleiter wie eine Glasfaser, verteilt sich das Licht entsprechend den Streu-, Absorptions- und Anisotropiekoeffizienten im Zahn. Setzt man den Lichtleiter wie die Glasfaser von okklusal senkrecht auf der approximalen Randleiste bestehend aus Zahnschmelz auf, breitet sich das Licht hauptsächlich im Zahnschmelz aus. Dennoch wird das Licht auch in gesundem Zahnschmelz durch geringe Streuung aus der ursprünglichen Strahlrichtung abgelenkt und kann daher von buccal oder lingual /palatinal beobachtet werden.

Der Zahn wird dabei von dem optischen Sensor wie CMOS-, CCD- oder InGaAs-Kamera erfasst, also gemessen, wobei die optische Achse des Sensors vorzugsweise senkrecht zur Längsachse des Zahns bzw. orthogonal zu dem Bereich ausgerichtet wird, der verändert ist, also insbesondere Karies aufweist. Aufgrund der erfindungsgemäßen Beauschlagung des Zahns mit Licht wird dieser aufgrund seiner Volumenstreuung zu einer Art selbstleuchtendem Objekt.

Dieser Effekt ist deutlich stärker ausgeprägt, wenn man das Licht an einer Stelle am Zahn injiziert, die nur eine geringe Schmelzschichtstärke aufweist. Im unter der Schmelzschicht liegenden Dentin wird das Licht viel stärker gestreut. In Abhängigkeit vom Injektionspunkt ergeben sich somit unterschiedliche Helligkeitsverteilungen im Zahn. Liegt unter dem Injektionspunkt eine dicke Schicht Zahnschmelz, kommt es nur zu einer geringen Streuung des Lichts in andere Raumrichtungen. Dies ist der Fall, wenn Licht auf die approximale Randleiste, also den durch den Zahnschmelz gebildeten Randbereich, parallel zur Approximalwand injiziert wird. Leitet man jedoch das Licht durch eine dünne Schmelzschicht ins Dentin, kommt es, sobald das Licht das Dentin erreicht hat, durch die dort höhere Streuung zu einer Art diffusen Hintergrundbeleuchtung des Zahnschmelzes.

Ersterer Fall eignet sich insbesondere für die Approximalkariesdetektion während sich die zweite Möglichkeit für Glattflächenkaries außerhalb des Approximalraums und eingeschränkt auch für Fissurenkaries eignet.

Karies verändert die optischen Eigenschaften der Zahnhartsubstanz. Im Zahnschmelz ändert sich in erster Linie der Streukoeffizient. Er erhöht sich signifikant durch die Bildung einer poröseren Mikrostruktur im Zahnschmelz bis hin zum Substanzverlust mit einer rauen Grenzfläche mit ebenfalls hoher Lichtstreuung zum verbleibenden Schmelz. Im Dentin ändern sich sowohl der Streu- als auch der Absorptionskoeffizient. Deutlich sichtbares Zeichen für die Änderung des Absorptionskoeffizienten ist die Braunverfärbung von kariösem Dentin.

Karies beginnt im Bereich der Zahnkrone immer im Zahnschmelz und arbeitet sich in Richtung Dentin vor. Damit entstehen die ersten optisch detektierbaren Änderungen im Zahnschmelz als Zonen mit lokal deutlich erhöhtem Streukoeffizienten. Erreicht die Karies das Dentin, kommt es entlang der Schmelz-Dentin-Grenze zu einer deutlichen Braunverfärbung durch Erhöhung des Absorptionskoeffizienten, insbesondere - jedoch nicht beschränkt - im blauen und grünen Spektralbereich.

Karies beginnt meistens an so genannten Prädilektionsstellen, unter anderem in Fissuren und unterhalb des Kontaktpunktes zweier Zähne. Letzterer Fall ist besonders schlecht visuell zu diagnostizieren, da er sich der direkten Beobachtung entzieht, da die Zahnfleischpapillen den Interproximalraum dreieckförmig abdecken und die benachbarten Zähne eng aneinander stehen. Es besteht jedoch die Möglichkeit, den Zahn von vestibulär bzw. lingual/buccal zu beobachten. Im Bereich des sichtbaren Lichts ist der Streukoeffizient von Schmelz bereits so hoch, dass aus der reinen Beobachtung unter Umgebungslicht und auch bei direkter Transillumination nur sehr begrenzte Aussagen zum Vorhandensein einer Karies getroffen werden können.

Um dennoch genügend Kontrast bei vestibulärer oder lingualer/bukkaler Beobachtung zu erreichen, wird der Injektionspunkt des Beleuchtungslichts variiert. Legt man den Injektionspunkt in Bezug zur Beobachtungsposition vor einen kariösen Bereich mit erhöhtem Streukoeffizienten, wird dieser Bereich hauptsächlich auf der Beobachtungsseite durch im Schmelz gestreutes Licht, aber auch partiell direkt und indirekt über am Dentin gestreutes Licht beleuchtet. Ein Bereich mit erhöhtem Streukoeffizient wird sich daher dem Beobachter (z. B. eines Bildaufnehmers bzw. Sensors) als heller Ort darstellen, weil das Licht an ihm stärker gestreut wird als in gesundem Zahnschmelz.

Liegt der Injektionspunkt in Bezug zur Beobachtungsposition hinter dem Bereich erhöhter Streuung, wird dieser Bereich von der Rückseite her beleuchtet. Dann erscheint dem Beobachter dieser Bereich dunkler, als wenn sich an der Stelle erhöhter Streuung gesunder Zahnschmelz befinden würde, da im Bereich erhöhter Streuung das Licht verstärkt vom Beobachtungspunkt weg gestreut wird. Speichert man jeweils ein Bild mit vor oder hinter der Karies liegend Lichtinjektionsstelle und subtrahiert diese voneinander, kommt es zu einer deutlichen Kontrastverbesserung.

In Weiterbildung dieses Gedankens kann ein ganzes Array von Fasern verwendet werden, um unterschiedliche Lichtinjektionspunkte zu ermöglichen. Die Lichtleitfasern eines Arrays können auch auf beiden Seiten eines Interproximalbereichs, also jeweils auf den gegenüberliegenden Randleisten zweier benachbarter Zähne positioniert werden. In diesem Fall kann ein Interproximalraum mit einer Messung gemessen werden. Ebenso können mehrere Kameras z. B. buccal und vestibulär gleichzeitig verwendet werden.

Eine Kamera kann auch eine Mikroendoskopoptik aufweisen, die an einer von der Lichtinjektionsstelle abweichenden Stelle auf den Zahn aufgesetzt wird.

Die Aufnahmen können mit einer Lichtwellenlänge sequentiell durchgeführt werden. Durch Wahl unterschiedlicher Wellenlängen für unterschiedliche Injektionspunkte kann jedoch auch parallel aufgenommen werden. Zur Dentinkariesdetektion können auch mehrere Wellenlängen über jeweils eine Faser appliziert werden, um durch Dentinkaries hervorgerufene Absorptionsunterschiede zu erkennen.

Die Erkennung von Glattflächenkaries erfolgt durch Nutzung des Dentins als selbstleuchtendes Objekt. Durch lokal begrenzte Lichtinjektion durch einen auf den Zahn aufgesetzten oder sich in unmittelbarer Nahe zur Zahnoberfläche befindlichen Lichtleiters kommt es zu keiner störenden Oberflächenreflexion, da diese in die Lichtleitfaser selbst zurückreflektiert wird. Das in den Zahn durch den Zahnschmelz eintretende Licht wird vom unter dem Zahnschmelz liegenden Dentin diffus und homogen gestreut und bildet so eine aus dem Zahn selbst kommende Hintergrundbeleuchtung. Liegt davor kariös veränderter Zahnschmelz, wird dieser als dunkler Fleck vor hellem Hintergrund deutlich sichtbar.

Das Erkennen von Schmelzrissen ist mit dieser Vorgehensweise ebenso möglich, da diese als Diskontinuität bei der Lichtleitung wirken und somit eine Beleuchtung vor dem Riss und hinter dem Riss in Bezug zur Beobachtungsposition deutlich unterschiedliche Lichtausbreitung im Zahn bewirkt.

Eine Kombination dieser Vorgehensweise mit einer Fluoreszenzdetektion ist ebenfalls möglich.

Selbstverständlich kann auch die Veränderung der Polarisationsebene von polarisiertem Licht durch die Zahnhartsubstanz zusätzlich genutzt werden.

Wird bevorzugterweise der zumindest eine Leiter unmittelbar auf den zu überprüfenden bzw. zu messenden Zahn gesetzt, so wird erwähntermaßen die erfindungsgemäße Lehre auch dann nicht verlassen, wenn ein geringer Abstand herrscht, der jedoch so gering zu wählen ist, dass von der Oberfläche reflektiertes Licht in den Lichtleiter wieder eintritt und nicht die Messung verfälscht.

Kann der Abstand gleich dem doppelten Durchmesser des Lichtleiterkerns sein, so sollte bevorzugterweise der Abstand gleich oder kleiner als der Lichtleiterkerndurchmesser selbst sein.

Erfindungsgemäß kann grundsätzlich die optische Achse des Sensors unter beliebigem Winkel zum Zahn ausgerichtet werden, um zu messen. Bevorzugterweise sollte die optische Achse jedoch senkrecht oder nahezu senkrecht zur Zahnlängsachse verlaufen.

Insbesondere zur interproximalen Karieserkennung, also Karies im Approximalraum zwischen benachbarten Zähnen, sollte die Stirnfläche des Lichtleiters, also die Lichtinjektionsstelle auf okklusalem durch den Zahnschmelz gebildeten Randbereich aufgesetzt bzw. auf diesen ausgerichtet werden. Dabei sollte die Strahlrichtung des austretenden Lichtes nach apical gerichtet sein.

Zur Lichtdetektion sollte bevorzugterweise eine CMOS-, CCD- oder InGaAs-Kamera verwendet werden. Bei Einsatz einer CCD-Kamera sollte diese einen in den Infrarotwellenbereich erweiterten Empfindlichkeitsbereich aufweisen.

Es besteht auch die Möglichkeit, dass dem optischen Sensor eine Mikroendoskopoptik vorgeschaltet wird, die an einer von der Lichtinjektionsposition verschiedenen Position auf den Zahn aufgesetzt oder in unmittelbarer Nähe der Zahnoberfläche angeordnet wird.

Zur Messung der Zahnveränderung im Approximalraum, also zur Interproximalkarieserkennung, sollte eine erste Messung bei einer Lichtbeaufschlagung des okklusalen Randbereichs in Bezug auf den optischen Sensor hinter der Zahnveränderung und eine zweite Messung bei einer Lichtbeaufschlagung der okklusalen Randleiste vor der Zahnveränderung durchgeführt werden. Die so aufgenommenen Bilder können verrechnet werden, um Rückschlüsse auf die Zahnveränderung zu ziehen.

Es besteht auch die Möglichkeit, dass zwei benachbarte Zähne gleichzeitig gemessen werden, wobei jedem Zahn zumindest ein Lichtleiter zugeordnet wird. Insbesondere können jedem Zahn zumindest zwei Lichtleiter zugeordnet werden, wobei in Bezug auf den optischen Sensor ein Lichtleiter vor und ein Lichtleiter hinter der Veränderung des Approximalraums der Zähne angeordnet werden.

Zur Ermittlung einer Veränderung des Zahns außerhalb des Approximalraums sollte der zumindest eine Lichtleiter auf den Kronenbereich des Zahns aufgesetzt bzw. auf diesen ausgerichtet werden, wobei die optische Achse des Sensors vorzugsweise orthogonal zur Veränderung des Zahnes auszurichten ist.

Der bzw. die Zähne sollten insbesondere mit einer Strahlung im Wellenlängenbereich beaufschlagt werden, die bevorzugt für die Lichtstreuung in der Schmelzschicht ist. Dabei sollte der Wellenlängenraum zwischen 300 nm und 1600 nm, vorzugsweise zwischen 350 nm und 1500 nm liegen. Sollen insbesondere Änderungen im Dentinbereich festgestellt werden, sind Messungen von Absorptionsänderungen von Vorteil. In diesem Fall sollte eine Beaufschlagung mit Licht in zumindest zwei Wellenlängenbereichen erfolgen, um eine spektrale Differenzmessung vornehmen zu können.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden bevorzugten Ausführungsbeispielen.

Es zeigen:
- Fig. 1: Prinzipdarstellungen von zwei Zähnen im saggitalen Schnitt,
- Fig. 2: eine der Fig. 1 entsprechende Darstellung mit im Approximalraum der Zähne vorhandener Karies und
- Fig. 3: die Darstellung gemäß Fig. 2 in Draufsicht.

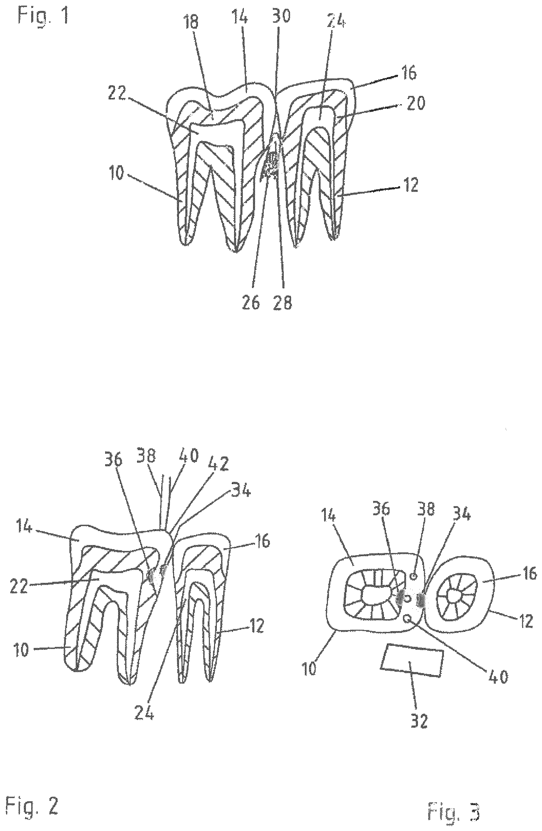

Der Fig. 1 ist ein prinzipieller Aufbau von Zähnen 10, 12 zu entnehmen, die im saggitalen Schnitt dargestellt sind. Jeder Zahn 10, 12 weist als äußere Schicht Zahnschmelz 14, 16 auf, die bis zu 3 mm stark sein kann. Unterhalb des Zahnschmelzes 14, 16 liegt das Dentin 18, 20, das den Zahnnerv (Pulpa) 22, 24 umgibt. Über dem Knochen 26 liegt das Zahnfleisch (Gingiva) 28, das zwischen den Zähnen 10, 12 eine dreieckförmige Geometrie aufweist, die den Zwischenraum zwischen den Zähnen 10, 12 schließt. Aus der Darstellung erkennt man des Weiteren, dass die Zähne 10, 12 sich in einem Kontaktbereich 30 berühren. Dieser auch als Approximalbereich bezeichnete Bereich zwischen den Zähnen 10, 12 unterliegt einer unmittelbaren visuellen Kontrolle nicht.

Karies bildet sich typischerweise unterhalb des Kontaktpunktes 30 zwischen den Zähnen 10, 12, wobei sich zunächst der Zahnschmelz 14, 16 kariös verändert. Schreitet die Karies weiter fort, wird auch das Dentin 18, 20 verändert.

Um unter Ausnutzung der Streu- und Absorptionseigenschaften von Zahnschmelz 14, 16 und Dentin 18, 20 Veränderung am Zahn 10, 12, insbesondere die Bildung von Karies festzustellen, wird erfindungsgemäß Licht lokal eng begrenzt in den bzw. die Zähne 10, 12 eingeleitet, quasi injiziert, woraufhin sich eine den optischen Eigenschaften des Zahns 10, 12 entsprechende Lichtverteilung im Zahnvolumen einstellt, die sodann von zumindest einem optischen Sensor wie Kamera 32 beobachtet bzw. gemessen werden kann.

In den Fig. 2 und 3 ist im Approximalbereich Karies prinzipiell eingezeichnet, wobei die Karies im Zahnschmelz 14 mit dem Bezugszeichen 34 und Karies im Dentin 18 mit dem Bezugszeichen 36 gekennzeichnet ist.

Um aufgrund der erfindungsgemäßen Lehre die Karies 34, 36 zu erfassen bzw. zu messen, wird über einen Lichtleiter 38 bzw. 40 Licht in den okklusalen und von dem Zahnschmelz 14 gebildeten Randbereich 42 eingeleitet. Hierzu wird der Lichtleiter 38, 40 mit seiner das Licht abstrahlenden Stirnfläche auf den Randbereich 42 - auch Randleiste genannt - aufgesetzt. Somit wird sichergestellt, dass von der Oberfläche des Zahns 10, 12 kein Licht reflektiert wird, welches von der Kamera 32 erfasst werden könnte, wodurch Messverfälschungen auftreten würden.

Die erfindungsgemäße Lehre wird jedoch auch dann nicht verlassen, wenn der Lichtleiter 38 bzw. 40 mit seiner Stirnfläche im geringen Abstand von der Randleiste 42 positioniert wird, wobei der Abstand so gering gehalten werden soll, dass das gesamte reflektierte Licht in den Lichtleiter 38 bzw. 40 hineinreflektiert wird.

Für die erfindungsgemäße Lehre reicht es aus, wenn einer der Lichtleiter 38, 40 zum Einsatz gelangt. Wird z. B. der Lichtleiter 40 okklusal und nach apical gerichtet auf die von dem Zahnschmelz 14 gebildete Randleiste 42 aufgesetzt, so wird das von dem Lichtleiter 40 abgegebene Licht hauptsächlich im Zahnschmelz 14 in Richtung der kariösen Stelle 34 geleitet und von dort in Richtung des optischen Sensors 32 gestreut, da sich der Lichtleiter 40 in Bezug auf die Kamera 32 vor der kariösen Veränderung 34 befindet. Wird der Lichtleiter 40 in die Position versetzt, die der des Lichtleiters 38 entspricht, also hinter der kariösen Stelle 34, so wird das eingeleitete Licht von der Kamera 32 weggestreut. Die in Richtung der Kamera 32 (Position des Lichtleiters 40) bzw. von der Kamera weg gestreute Strahlung (Position des Lichtleiters 38) korreliert sodann mit der Größe des kariös veränderten Volumens.

Der kariöse Bereich 36, der im Dentin 18 verläuft, weist nicht nur eine entsprechende Veränderung im Zahnschmelz 14 auf, wie dies zuvor erläutert worden ist, sondern gleichzeitig sind auch das Streu- und Absorptionsverhalten des Dentins 18 verändert worden. Durch die zusätzliche Veränderung des Absorptionskoeffizienten ergeben sich Differenzierungsmöglichkeiten hinsichtlich des Erreichens von Dentin 18 bei Beleuchtung mit mindestens zwei verschiedenen Wellenlängen, die derart auf die Veränderung des Absorptionsverhaltens des Dentins 18 aufgrund des kariösen Bereichs 36 ausgelegt sind, dass sich bei einer Wellenlänge die Absorption bei Vorhandensein des kariösen Bereichs 36 nur wenig ändert, während sich bei dem anderen Wellenlängenbereich die Absorption signifikant von gesundem Dentin 18 unterscheidet.

Zu erwähnen ist, dass das Licht in den Zahn 10, 12 grundsätzlich über einen einzigen Lichtleiter wie Glasfaser eingeleitet werden sollte. Die Erfindung wird jedoch dann nicht verlassen, wenn Licht über zwei oder drei Leiter injiziert wird. In diesem Fall könnte jedoch der zu messende Kontrast abgeschwächt sein.

## Patentansprüche

1. Verfahren zum Erkennen von Veränderungen an zumindest einem Zahn (10, 12) im Dentin- und/oder Zahnschmelzbereich, wie Karies (34, 36) oder Zahnschmelzriss, durch Beaufschlagen des zumindest einen Zahns mit aus zumindest einem Lichtleiter (38, 40) über dessen Stirnfläche austretendem Licht sowie Erfassen zumindest eines Bereichs des Zahns mittels eines optischen Sensors (32), wie Kamera, und anschließendes Auswerten von von dem Sensor ermittelten Bildern, wobei zum Beaufschlagen des zumindest einen Zahns mit dem Licht des zumindest einen Lichtleiters die Stirnfläche des Lichtleiters auf den Zahn aufgesetzt oder im Abstand a mit a ≤ 2d mit d = Lichtleiterkerndurchmesser zur Oberfläche des Zahns derart positioniert wird, dass von der Zahnoberfläche reflektiertes Licht ausschließlich in den Lichtleiter hinein zurückreflektiert wird, und wobei mit dem optischen Sensor aus dem Zahn austretendes Streulicht gemessen wird,
**dadurch gekennzeichnet,**
**dass** zumindest ein erstes Bild des zumindest einen Zahns (10, 12) in Bezug auf die Veränderung bei Lichtbeaufschlagung vor der Veränderung des zumindest einen Zahns (10, 12) aufgenommen wird und dass zumindest ein zweites Bild des zumindest einen Zahns bei Lichtbeaufschlagung hinter der Veränderung aufgenommen wird, jeweils in Bezug zur Sensorposition, und dass das zumindest eine erste Bild mit dem zumindest einen zweiten Bild verrechnet wird.

2. Verfahren nach Anspruch 1,
**dadurch gekennzeichnet,**
**dass** die Stirnfläche des Lichtleiters (38, 40) mit dem Abstand a ≤ d zur Oberfläche des Zahns (10, 12) positioniert wird.

3. Verfahren nach zumindest einem der vorhergehenden Ansprüche,
**dadurch gekennzeichnet,**
**dass** Hauptstrahlrichtung des aus dem zumindest einen Lichtleiter (38, 40) austretenden Lichts nach apical gerichtet wird.

4. Verfahren nach zumindest einem der vorhergehenden Ansprüche,
**dadurch gekennzeichnet,**
**dass** die Stirnfläche des Lichtleiters (38, 40) auf okklusalen durch den Zahnschmelz (14, 16) gebildeten Randbereich (42) aufgesetzt bzw. ausgerichtet wird.

5. Verfahren nach zumindest einem der vorhergehenden Ansprüche,
**dadurch gekennzeichnet,**
**dass** als optischer Sensor (32) zumindest eine CMOS-, CCD- oder InGaAs-Kamera oder eine CCD-Kamera mit in den Infrarot-Wellenlängenbereich erweitertem Empfindlichkeitsbereich verwendet wird.

6. Verfahren nach zumindest einem der vorhergehenden Ansprüche,
**dadurch gekennzeichnet,**
**dass** dem optischen Sensor eine Mikroendoskopoptik vorgeschaltet wird, die auf den zumindest einen Zahn aufgesetzt oder in unmittelbarer Nähe der Zahnoberfläche positioniert wird.

7. Verfahren nach zumindest einem der vorhergehenden Ansprüche,
**dadurch gekennzeichnet,**
**dass** zur Messung einer Zahnveränderung im Approximalraum oder zur Interproximalkarieserkennung eine erste Messung bei einer Lichtbeaufschlagung des okklusalen Randbereichs in Bezug auf den optischen Sensor (32) hinter der Zahnveränderung und eine zweite Messung bei einer Lichtbeaufschlagung des okklusalen Randbereichs vor der Zahnveränderung durchgeführt wird.

8. Verfahren nach zumindest einem der vorhergehenden Ansprüche,
**dadurch gekennzeichnet,**
**dass** ein Array von Lichtleitern (38, 40) dem zumindest einen Zahn (10, 12) zugeordnet wird und dass zum Erfassen bzw. Messen der Zahnveränderung ein oder mehrere Lichtleiter des Arrays mit Licht gleichen Wellenlängenbereichs beaufschlagt werden.

9. Verfahren nach zumindest einem der Ansprüche 1 bis 7,
**dadurch gekennzeichnet,**
**dass** ein Array von Lichtleitern dem zumindest einen Zahn zugeordnet wird und zum Erfassen der Zahnveränderung die Lichtleiter des Arrays gleichzeitig mit Licht beaufschlagt werden, wobei in jeden einzelnen Lichtleiter Licht in einem Wellenlängenbereich eingekoppelt wird, der von dem der anderen Lichtleiter abweicht.

10. Verfahren nach zumindest einem der vorhergehenden Ansprüche,
**dadurch gekennzeichnet,**
**dass** zwei benachbarte Zähne (10, 12) gleichzeitig gemessen werden, wobei jedem Zahn zumindest ein Lichtleiter (38, 40) zugeordnet wird.

11. Verfahren nach zumindest einem der vorhergehenden Ansprüche,
**dadurch gekennzeichnet,**
**dass** jedem Zahn (10, 12) zumindest zwei Lichtleiter (38, 40) zugeordnet werden, wobei in Bezug auf den optischen Sensor (32) ein Lichtleiter vor und ein Lichtleiter hinter der Zahnveränderung im Approximalraum der Zähne angeordnet werden.

12. Verfahren nach zumindest einem der vorhergehenden Ansprüche,
**dadurch gekennzeichnet,**
**dass** zur Ermittlung einer Veränderung des zumindest einen Zahns (10, 12) außerhalb des Approximalraums zumindest ein Lichtleiter auf den Kronenbereich des Zahns aufgesetzt bzw. auf diesen ausgerichtet wird und dass der optische Sensor (32) mit seiner optischen Achse vorzugsweise orthogonal zur Veränderung des Zahns ausgerichtet wird.

13. Verfahren nach zumindest einem der vorhergehenden Ansprüche,
**dadurch gekennzeichnet,**
**dass** der zumindest eine Zahn (10, 12) mit Licht mit einem Wellenlängenbereich Δλ beaufschlagt wird, in dem vorrangig Lichtstreuung in dem Zahnschmelz auftritt, wobei insbesondere der Zahn mit Licht im Wellenlängenraum zwischen 300 nm und 1600 nm, vorzugsweise zwischen 350 nm und 1500 nm beaufschlagt wird.

14. Verfahren nach zumindest einem der vorhergehenden Ansprüche,
**dadurch gekennzeichnet,**
**dass** zur Erfassung der Veränderung des zumindest einen Zahns (10, 12) im Dentin (18, 20) spektrale Differenzmessungen durchgeführt werden.

## Claims

1. A method for detecting alterations on at least one tooth in the dentine and tooth enamel area such as caries (3, 36) or cracks in the enamel, comprising:
impinging at least one tooth with light escaping from a front surface of the at least one light guide (38, 40),
recording at least one area of the at least one tooth by the an optical sensor, such as camera, and subsequently assessing the images determined by the optical sensor;
wherein, in order to impinge the light from the at least one light guide to the at least one tooth, the front surface of the light guide is placed on the at least one tooth, or positioned at a distance a, with a≦2d and d=light guide core diameter, from the surface of the at least one tooth, such that light reflected by the at least one tooth surface is reflected at least substantially exclusively back into the light guide, and the scattered light escaping from the at least one tooth is measured with the optical sensor,
**characterized in,**
**that** at least a first image of the at least one tooth (10, 12) with respect to the alteration is recorded with light application in front of the alteration of the at least one tooth (10, 12), and at least one second image of the at least one tooth is recorded with light application behind the alteration, each with respect to the sensor position, and that the at least one first image is processed against the at least second image.

2. The method according to claim 1,
**characterized in**
**that** the front of the light guide (38, 40) is positioned at a distance a≦d from to a surface of the tooth (10, 12).

3. The method according to at least one of the proceeding claims,
**characterized in**
**that** a main beam direction of the light escaping from the at least one light guide (38, 40) is directed toward the apical side.

4. The method according to at least one of the proceeding claims,
**characterized in**
**that** the front surface of the light guide (38, 40) is placed on or aligned with an occlusal boundary area formed by the tooth enamel.

5. The method according to at least one of the proceeding claims,
**characterized in**
**that** at least one CMOS, CCD, or InGaAs camera with extended sensitivity in the infrared range is used as the optical sensor.

6. The method according to at least one of the proceeding claims,
**characterized in**
**that** a microendoscope optic is arranged in front of the optical sensor and is placed on the at least one tooth or is positioned in the direct vicinity of the tooth surface.

7. The method according to at least one of the proceeding claims,
**characterized in**
**that** a first measurement is carried out with light applied to an occlusal boundary area with respect to the optical sensor (32) behind the tooth alteration, and a second measurement is carried out with light applied to an occlusal marginal ridge in front of the tooth alteration, in order to measure a tooth alteration in an approximal space or for interproximal caries detection.

8. The method according to at least one of the proceeding claims,
**characterized in**
**that** an array of light guides (38, 40) is assigned to the at least one tooth (10, 12) and that one or more light guides of the array are subjected to light having a same wavelength range in order to record or measure the tooth alteration.

9. The method according to at least one of the claims 1 to 7,
**characterized in**
**that** an array of light guides is assigned to the at least one tooth in that light is simultaneously applied to the light guides of the array in order to record the tooth alteration, wherein into each individual light guide is coupled, having a wavelength range that is different from that of the other light guides.

10. The method according to at least one of the proceeding claims,
**characterized in**
**that** two adjacent teeth (10, 12) are simultaneously measured, wherein at least one light guide (38, 40) is associated with each tooth.

11. The method according to at least one of the proceeding claims,
**characterized in**
**that** at least two light guides (38, 40) are associated with the at least one tooth (10, 12), wherein one light guide is arranged in front and one light guide is arranged behind the tooth alteration with reference to the optical sensor (32) in an approximal space of the teeth.

12. The method according to at least one of the proceeding claims,
**characterized in**
**that** in order to determine an alteration of the at least one tooth (10, 12) outside an approximal space, at least one light guide is placed on an area of the at least one tooth, or is aligned therewith, and the optical sensor (32) is aligned with the optical axis thereof, substantially orthogonally with respect to the alteration of the at least one tooth.

13. The method according to at least one of the proceeding claims,
**characterized in**
**that** the at least one tooth (10, 12) is subjected to light having a wavelength range Δλ, in which preferentially light scattering occurs in the tooth enamel, wherein in particular the tooth is subjected to light in the wavelength range between 300 nm and 1600 nm, preferably in the wavelength range between 350 nm and 1500 nm.

14. The method according to at least one of the proceeding claims,
**characterized in**
**that** spectral differential measurements are carried out in the dentine (18, 20) in order to image the alteration of the at least one tooth (10, 12).

## Revendications

1. Procédé pour détecter des altérations sur au moins une dent (10, 12) dans la région de la dentine et/ou de l'émail, telle qu'une carie (34, 36) ou une fissure de l'émail dentaire, en appliquant sur au moins une dent une lumière sortant d'au moins un guide de lumière (38, 40) par sa surface frontale ainsi que pour acquérir au moyen d'un capteur optique (32), tel qu'une caméra, au moins une partie de la dent puis évaluer les images transmises par le capteur,
sachant que pour appliquer la lumière de l'au moins un guide de lumière sur l'au moins une dent, la surface frontale du guide de lumière est placée sur la dent ou à une distance a telle que a ≤ 2d où d = diamètre du noyau du guide de lumière par rapport à la surface de la dent, de telle manière que la lumière réfléchie par la surface de la dent est renvoyée exclusivement dans le guide de lumière, et que la lumière diffusée sortant de la dent est mesurée par le capteur optique,
**caractérisé en ce**
**qu'**au moins une première image de l'au moins une dent (10, 12) est enregistrée, en rapport avec l'altération, en appliquant la lumière devant l'altération de l'au moins une dent (10, 12), et en ce qu'au moins une seconde image de l'au moins une dent est enregistrée en appliquant la lumière derrière l'altération, respectivement par rapport à la position du capteur, et que l'au moins une première image est traitée avec l'au moins une seconde image.

2. Procédé selon la revendication 1,
**caractérisé en ce**
**que** la surface frontale du guide de lumière (38, 40) est positionnée à la distance a ≤ d de la surface de la dent (10, 12).

3. Procédé selon au moins une des revendications précédentes,
**caractérisé en ce**
**que** la direction du faisceau principal de la lumière sortant de l'au moins un guide de lumière (38, 40) est dirigée du côté apical.

4. Procédé selon au moins une des revendications précédentes,
**caractérisé en ce**
**que** la surface frontale du guide de lumière (38, 40) est placée ou alignée sur la zone de bord occlusale (42) formée par l'émail dentaire (14, 16).

5. Procédé selon au moins une des revendications précédentes,
**caractérisé en ce**
**qu'**au moins une caméra CMOS, CCD ou InGaAs ou une caméra CCD avec une plage de sensibilité étendue dans la plage de longueur d'onde infrarouge est utilisée comme capteur optique (32).

6. Procédé selon au moins une des revendications précédentes,
**caractérisé en ce**
**que** le capteur optique est précédé d'un système optique de microendoscope qui est placé sur l'au moins une dent ou à proximité immédiate de la surface de la dent.

7. Procédé selon au moins une des revendications précédentes,
**caractérisé en ce**
**que**, pour mesurer une altération dentaire dans la niche interproximale ou pour détecter une carie interproximale, une première mesure est effectuée lorsque la zone de bord occlusale est exposée à une lumière appliquée derrière l'altération dentaire par rapport au capteur optique (32) et une seconde mesure est effectuée lorsque la zone de bord occlusale est exposée à une lumière appliquée devant l'altération dentaire.

8. Procédé selon au moins une des revendications précédentes,
**caractérisé en ce**
**qu'**un faisceau de guides de lumière (38, 40) est affecté à l'au moins une dent (10, 12), et en ce qu'un ou plusieurs guides de lumière de cet ensemble sont alimentés en une lumière de même longueur d'onde afin d'acquérir ou mesurer l'altération dentaire.

9. Procédé selon au moins une des revendications 1 à 7,
**caractérisé en ce**
**qu'**un faisceau de guides de lumière est affecté à l'au moins une dent et que les guides de lumière de ce faisceau sont alimentés simultanément en lumière afin d'acquérir l'altération dentaire, la lumière étant couplée dans chaque guide de lumière individuel dans une plage de longueurs d'onde qui diffère de celle des autres guides de lumière.

10. Procédé selon au moins une des revendications précédentes,
**caractérisé en ce**
**que** deux dents voisines (10, 12) sont mesurées simultanément, sachant qu'au moins un guide de lumière (38, 40) est associé à chaque dent.

11. Procédé selon au moins une des revendications précédentes,
**caractérisé en ce**
**qu'**au moins deux guides de lumière (38, 40) sont affectés à chaque dent (10, 12), sachant qu'un guide de lumière est disposé devant et un guide de lumière derrière l'altération dentaire située dans la niche interproximale des dents, par rapport au capteur optique (32).

12. Procédé selon au moins une des revendications précédentes,
**caractérisé en ce**
**que**, pour déterminer une altération d'au moins une dent (10, 12) à l'extérieur de la niche interproximale, au moins un guide de lumière est placé sur ou aligné avec la zone de la couronne de la dent, et en ce que l'axe optique du capteur optique (32) est orienté de préférence orthogonalement par rapport à l'altération dentaire.

13. Procédé selon au moins une des revendications précédentes,
**caractérisé en ce**
**que** l'au moins une dent (10, 12) est soumise à une lumière présentant une plage de longueurs d'onde *Δλ* dans laquelle la diffusion de la lumière se produit principalement dans l'émail de la dent, sachant en particulier que la dent est soumise à une lumière dans la plage de longueurs d'onde entre 300 nm et 1600 nm, de préférence entre 350 nm et 1500 nm.

14. Procédé selon au moins une des revendications précédentes,
**caractérisé en ce**
**que** des mesures de différence spectrale sont réalisées dans la dentine (18, 20) afin d'acquérir l'altération dans au moins une dent (10, 12).
